# EUROPEAN PATENT SPECIFICATION

(11) **EP 1 786 548 B1**
(45) Date of publication and mention of the grant of the patent: **24.12.2014**
(21) Application number: 05756680.4
(22) Date of filing: 28.06.2005
(51) Int. Cl.: B01J 2/22, A61K 9/16, A61K 9/20, A61K 31/54

(54) **GRANULES COMPRISING A ß-LACTAM ANTIBIOTIC**
BETA-LACTAM-ANTIBIOTIKUM ENTHALTENDES PRODUKT
PRODUIT CONTENANT UN ANTIBITIQUE BÊTA-LACTAME

(30) Priority: 30.06.2004 EP 04076894
(43) Date of publication of application: 23.05.2007
(73) Proprietor: DSM Sinochem Pharmaceuticals Netherlands B.V., 2613 AX Delft (NL)
(72) Inventor: RIJKERS, Marinus, Petrus, Wilhelmus, Maria, NL-6181 DH Elsloo (NL); DUCHATEAU, Alexander, Lucia, Leonardus, B-3620 Lanaken (BE); MOMMERS, Johannes, Helena, Michael, NL-6142 CA Einighausen (NL); BOESTEN, Jozef, Maria, Mathias, NL-6336 WC Hulsberg (NL)
(74) Representative: Misset, Onno
(86) International application number: PCT/EP2005/053036
(87) International publication number: WO 2006/003152

(56) References cited:
- WO-A-99/11261
- US-A- 4 134 943
- US-B1- 6 358 526

## Description

The present invention relates to granules comprising a β-lactam antibiotic, to a process for the preparation thereof and to a use of an apparatus for preparing the granules comprising the β-lactam antibiotic.

The preparation of a β-lactam antibiotic typically involves obtaining the β-lactam antibiotic as a crystalline powder, e.g. by crystallizing the β-lactam antibiotic from a solution, and drying the resulting crystals resulting in the powder. When improved physical properties are desired, e.g. bulk density or flowability, the powder may be compressed, e.g. by roller compacting to form granules comprising compressed powder. Roller compacting of a β-lactam antibiotic is e.g. described in WO-A-9911261.

It was found that the known process for the preparation of granules comprising a β-lactam antibiotic in compressed form results in a product having an unpleasant smell. It is an object of the invention to provide granules comprising having no smell or a smell that is at least less intensive.

This object is achieved by providing a process for preparing granules comprising a ß-lactam antibiotic according to the appended claims. The process is described in detail further below.

The granules obtainable by the process of the invention, wherein C_{H2S(72h)} < 50 µl of H₂S gas per kg of β-lactam antibiotic, wherein C_{H2S(72h)} is the volume of H₂S gas above said granules per kg of said β-lactam antibiotic, when a sample of between 3.5 and 4.5 g of said granules is kept in a closed container having a volume of 20 ml at a temperature of 22 °C during 72 hours at atmospheric pressure (1 bar).

Preferably, the granules comprising a β-lactam antibiotic according to the invention, have a C_{H2S(72h)} < 40 µl of H₂S gas per kg of β-lactam antibiotic, preferably C_{H2S(72h)} < 30 µl of H₂S gas per kg of β-lactam antibiotic, preferably C_{H2S(72h)} < 25 µl of H₂S gas per kg of β-lactam antibiotic, preferably C_{H2S(72h)} < 20 of H₂S gas per kg of β-lactam antibiotic, when a sample of between 3.5 and 4.5 g of said granules is kept in a closed container having a volume of 20 ml at a temperature of 22 °C during 72 hours at atmospheric pressure (1 bar). In an embodiment, the granules comprising a β-lactam antibiotic according to the invention have a C_{H2S(72h)} > 1 µl of H₂S gas per kg of β-lactam antibiotic.

As used herein C_{H2S(72h)} is determined under the following conditions: a sample of between 3.5 and 4.5 gram of said granules is kept in a closed container (volume of 20 ml) at a temperature of 22 °C during 72 hours at atmospheric pressure (1 bar). After said 72 hours a sample of air is taken from the container and analysed by gas chromatography to determine the volume fraction of H₂S in said sample of air. Said volume fraction of H₂S gas is multiplied by the gas phase volume above the sample (i.e. volume of the container, i.e. 20 ml, minus the volume of the sample) resulting in the volume of H₂S gas in the container. The calculated value of said volume of H₂S gas in the containe is divided by the weight of the sample, resulting in C_{H2S(72h)}.

The disclosure also provides granules comprising a β-lactam antibiotic obtainable by the process of the invention, wherein C_{H2S(3h)} < 10 µl of H₂S gas per kg of β-lactam antibiotic, wherein C_{H2S(3h)} is the volume of H₂S gas above said granules per kg of said β-lactam antibiotic, when a sample of between 3.5 and 4.5 g of said granules is kept in a closed container having a volume of 20 ml at a temperature of 22 °C during 3 hours at atmospheric pressure (1 bar).

In the latter aspect of the disclosure, the C_{H2S(3h)} is determined under the following conditions: a sample of between 3.5 and 4.5 gram of said granules is kept in a closed container (volume of 20 ml) at a temperature of 22 °C during 3 hours at atmospheric pressure (1 bar). After said 3 hours a sample of air is taken from the container and analysed by gas chromatography to determine the volume fraction of H₂S in said sample of air. Said volume fraction of H₂S gas is multiplied by the gas phase volume above the sample (i.e. volume of the container, i.e. 20 ml, minus the volume of the sample) resulting in the volume of H₂S gas in the container. The calculated value of said volume of H₂S gas in the container is divided by the weight of the sample, resulting in C_{H2S(3h)}·

In a preferred aspect, the disclosure provides granules comprising a β-lactam antibiotic obtainable by the process of the invention, wherein C_{H2S(3h)} < 9 µl of H₂S gas per kg of β-lactam antibiotic, preferably C_{H2S(3h)} < 8 µl of H₂S gas per kg of p-lactam antibiotic, preferably C_{H2S(3h)} < 7 µl of H₂S gas per kg of β-lactam antibiotic, preferably C_{H2S(3h)} < 6 µl of H₂S gas per kg of β-lactam antibiotic, preferably C_{H2S(3h)} < 5 µl of H₂S gas per kg of β-lactam antibiotic when a sample of between 3.5 and 4.5 g of said granules is kept in a closed container having a volume of 20 ml at a temperature of 22 °C during 3 hours at atmospheric pressure (1 bar). In an embodiment, the granules comprising a β-lactam antibiotic according to the invention have a C_{H2S(3h)} > 1 µl of H₂S gas per kg of β-lactam antibiotic.

Granules obtained by a process according to the invention may comprise auxiliaries or may be free of auxiliaries. Granules according to the invention may comprise compressed β-lactam antibiotic, for instance β-lactam antibiotic compressed by roller compacting. Granules according to the invention are preferably obtained by roller compacting. The granules according to the invention have a bulk density of between 0.4 and 1.0 g/ml, for instance between 0.45 and 0.8 g/ml. As used herein, bulk density is preferably determined using USP 24, method I, (page 1913). Preferably, bulk density is determined using method Eur. Ph. 5.0, section 2.9.15.

As auxiliaries may for instance be used fillers, dry binders, disintegrants, wetting agents, wet binders, lubricants, flow agents and the like. Examples of auxiliaries are lactose, starches, bentonite, calcium carbonate, mannitol, microcrystalline cellulose, polysorbate, sodium lauryl sulphate, carboxymethylcellulose Na, sodium alginate, magnesium stearate, silicon dioxid, talc. Preferably, the granules according to the invention are free of auxiliaries.

The invention provides a process for preparing granules according to the invention.

The invention provides a process for preparing granules comprising a β-lactam antibiotic, said process comprising feeding said β-lactam antibiotic to a roller compactor to form compacts, size reducing, e.g. milling the compacts to produce granules, wherein the temperature of the β-lactam antibiotic that is fed to the roller compactor is below 20°C and wherein C_{H2S(72h)} < 50 µl of H₂S gas per kg of β-lactam antibiotic, preferably C_{H2S(72h)} < 40 µl of H₂S gas per kg of β-lactam antibiotic, preferably C_{H2S(72h)} < 30 µl of H₂S gas per kg of β-lactam antibiotic, preferably C_{H2S(72h)} < 25 µl of H₂S gas per kg of β-lactam antibiotic, preferably C_{H2S(72h)} < 20 µl of H₂S gas per kg of β-lactam antibiotic, when a sample of between 3.5 and 4.5 g of said granules is kept in a closed container having a volume of 20 ml at a temperature of 22 °C during 72 hours at atmospheric pressure (1 bar).

In another embodiment, the invention provides a process for preparing granules comprising a β-lactam antibiotic, said process comprising feeding said β-lactam antibiotic to a roller compactor to form compacts, size reducing, e.g. milling the compacts to produce granules, wherein the temperature of the β-lactam antibiotic that is fed to the roller compactor is below 20°C and wherein C_{H2S(3h)} < 10 µl of H₂S gas per kg of β-lactam antibiotic, preferably C_{H2S(3h)} < 9 µl of H₂S gas per kg of β-lactam antibiotic, preferably C_{H2S(3h)} < 8 µl of H₂S gas per kg of β-lactam antibiotic, preferably C_{H2S(3h)} < 7 µl of H₂S gas per kg of β-lactam antibiotic, preferably C_{H2S(3h)} < 6 µl of H₂S gas per kg of β-lactam antibiotic, preferably C_{H2S(3h)} < 5 µl of H₂S gas per kg of β-lactam antibiotic when a sample of between 3.5 and 4.5 g of said granules is kept in a closed container having a volume of 20 ml at a temperature of 22 °C during 3 hours at atmospheric pressure (1 bar).

Surprisingly it was found that by cooling the antibiotic to a temperature below 20°C prior to feeding to the roller compactor decreases C_{H2S(72h)} and C_{H2S(3h)}.

In an embodiment, the invention provides a process for preparing granules comprising a β-lactam antibiotic, said process comprising feeding said β-lactam antibiotic to a roller compactor to form compacts, size reducing, e.g. milling the compacts to produce granules, wherein the said β-lactam antibiotic is cooled prior to said feeding.

In another embodiment, the invention provides a process for preparing granules comprising a β-lactam antibiotic, according to the appended claims said process comprising feeding said β-lactam antibiotic to a roller compactor to form compacts, size reducing, e.g. milling the compacts to produce granules, wherein the temperature of the β-lactam antibiotic that is fed to said roller compactor is below 18 °C, preferably below 15 °C.

The β-lactam antibiotic is preferably fed to the roller compactor as a crystalline powder of the β-lactam antibiotic, preferably without auxiliaries. However, it is also possible to feed a mixture comprising a crystalline powder and auxiliaries to the roller compactor.

As auxiliaries may for instance be used fillers, dry binders, disintegrants, wetting agents, wet binders, lubricants, flow agents and the like. Examples of auxiliaries are lactose, starches, bentonite, calcium carbonate, mannitol, microcrystalline cellulose, polysorbate, sodium lauryl sulphate, carboxymethylcellulose Na, sodium alginate, magnesium stearate, silicon dioxid, talc.

The roller compactor may be operated at any suitable roller pressure, for instance between 10 and 250 kN, for instance between 50-200 kN.

The disclosure also provides a process for compressing a β-lactam antibiotic, said process comprising feeding said β-lactam antibiotic to a step in which the β-lactam antibiotic is compressed to form compressed β-lactam antibiotic, wherein the temperature of the β-lactam antibiotic that is fed to said step is sufficiently low that C_{H2S(72h)} < 50 µl of H₂S gas per kg of β-lactam antibiotic, preferably C_{H2S(72h)} < 40 µl of H₂S gas per kg of β-lactam antibiotic, preferably C_{H2S(72h)} < 30 µl of H₂S gas per kg of β-lactam antibiotic, preferably C_{H2S(72h)} < 25 µl of H₂S gas per kg of β-lactam antibiotic, C_{H2S(72h)} < 20 µl of H₂S gas per kg of β-lactam antibiotic, when a sample of between 3.5 and 4.5 g of said granules is kept in a closed container having a volume of 20 ml at a temperature of 22 °C during 72 hours at atmospheric pressure (1 bar).

The disclosure also provides a process for compressing a β-lactam antibiotic, said process comprising feeding said β-lactam antibiotic to a step in which the β-lactam antibiotic is compressed to form compressed β-lactam antibiotic, wherein the temperature of the β-lactam antibiotic that is fed to said step is sufficiently low that C_{H2S(3h)} < 10 µl of H₂S gas per kg of β-lactam antibiotic, preferably C_{H2S(3h)} < 9 µl of H₂S gas per kg of β-lactam antibiotic, preferably C_{H2S(3h)} < 8 µl of H₂S gas per kg of β-lactam antibiotic, C_{H2S(3h)} < 7 µl of H₂S gas per kg of β-lactam antibiotic, preferably C_{H2S(3h)} < 6 µl of H₂S gas per kg of β-lactam antibiotic, preferably C_{H2S(3h)} < 5 µl of H₂S gas per kg of β-lactam antibiotic, when a sample of between 3.5 and 4.5 g of said granules is kept in a closed container having a volume of 20 ml at a temperature of 22 °C during 3 hours at atmospheric pressure (1 bar).

In an aspect, the process comprises cooling the β-lactam antibiotic prior to said feeding.

In an aspect, the process comprises feeding said β-lactam antibiotic to a step in which the β-lactam antibiotic is compressed to form compressed β-lactam antibiotic, wherein the β-lactam antibiotic is cooled prior to said feeding.

The temperature of the β-lactam antibiotic that is fed to said step is below 20 °C, preferably below 18 °C, more preferably below 15 °C.

The invention also provides the use of an apparatus for the preparation of the granules obtained by the process of the invention comprising
(i) a cooler for cooling an antibiotic; and
(ii) a means for compressing the cooled antibiotic,
wherein said (i) cooler is arranged such that the antibiotic is cooled to a temperature below 20°C prior to feeding the antibiotic to (ii) the means for compressing the antibiotic.

Preferably said means for compressing the antibiotic is a roller compactor. Preferably, said apparatus further comprises a dryer for drying the β-lactam antibiotic, said dryer arranged such that the dried antibiotic can be fed to the cooler.

The β-lactam antibiotic is not limited to a specific type of β-lactam antibiotic. It may for instance be a penicillin, for instance ampicillin or amoxicillin, or a cephalosporin, for instance cephalexin, cefadroxil, cephradin, or cefalcor.

Cephalexin may be in any suitable form, for instance in the form of a hydrate, for instance cephalexin monohydrate.

Cefadroxil may be in any suitable form, for instance in the form of a hydrate, for instance cefadroxil monohydrate.

Cephradin may be in any suitable form, for instance in the form of a hydrate, for instance cephradin monohydrate.

Cefaclor may be in any suitable form, for instance in the form of a hydrate, for instance cefaclor monohydrate.

Amoxicillin may be in any suitable form, for instance in the form of a hydrate, for instance amoxicillin trihydrate.

Ampicillin may be in any suitable form, for instance in the form of a hydrate, for instance ampicillin trihydrate.

The β-lactam antiobiotic may be prepared in any suitable process known in the art, for instance using a chemical process or an enzymatic process.

### EXAMPLES

### Materials

In comparative experiment A and example 1, cephalexin was prepared and recovered using the process as described in WO-A-9623796. The cephalexin (monohydrate) crystals obtained were washed with water and subsequently with a water-acetone mixture containing 80 vol.% of acetone. The resulting wet cake contained 8 wt.% of free water and 8 wt.% of acetone.

In addition one sample of cephalexin granules in comparative experiment A was obtained from company A.

In comparative experiment B, a sample of granules of cephradin was obtained from company B.

In example 2, cephradine was prepared and recovered according to the method as described in WO 2005/003367, using PenG acylase mutant Phe-24-Ala. The cephradine hydrate crystals obtained, were washed with water and subsequently with a water-acetone mixture containing 80 vol.% of acetone. The resulting wet cake contained 8 wt.% of free water and 8 wt.% of acetone.

### Comparative experiment A

The cephalexin wet cake was dried using a Vacuum Paddle dryer type SHV-3000 supplied by Bachiller S.A., Spain. The dryer was charged with 600 kg cephalexin wet cake produced as described above, containing 8 wt.% of free water and 8 wt.% of acetone. The walls were heated at a temperature of 70 °C (product temperature 40 °C). The final pressure was 20 mbar. During drying the wet cake was stirred at a speed of 7 rpm. After 2 hours and 40 minutes of drying the product was discharged. The water content was 5.2 wt.% (Karl Fisher).

The resulting powder, having a temperature between 20-25 °C, was fed to a roller compactor produced by Hosokawa-Bepex, type K200/100 operated at a roller speed of 12 rpm and a roller pressure of 130 kN. The resulting compacted product was crushed to obtain granules having a bulk density above 0.45 g/ml and a tapped density above 0.75 g/ml. The densities were determined using method Eur. Ph. 5.0, section 2.9.15 (with the difference that a 100 ml cylinder was used).

The resulting product was analysed for the H₂S content using a HP 6890 gas chromatograph, and a Supelco SPB-1 sulfur, 30 m x 0.32 mm x 4.00 column. 3 reference experiments were carried using gases containing known volume concentrations of H₂S in N₂: (0.5 vol ppm, 1.5 ppm, and 5.6 ppm). Using these reference experiments, a calibration curve was constructed.

### A.1. Determination C_{H2S(3h)}

After this the C_{H2S(3h)} of the cephalexin granules was determined.

A sample (4.04 gram), was introduced into a vial having a volume of 20 ml. The sample was equilibrated at ambient temperature (22 °C) for 3 hours. After said 3 hours a sample of air (300 µl injection volume) from the vial was analysed. The H₂S vol ppm in said sample was 3.2 ppm. The volume of the gas phase above the sample was 14.5 ml (i.e. sample volume was 5.5 ml). Hence, C_{H2S(3h)} = 11 µl of H₂S gas per kg of β-lactam antibiotic. This experiment was repeated 3 times, resulting in an average value for C_{H2S(3h)} of 10 µl of H₂S gas per kg of β-lactam antibiotic.

### A.2. Determination C_{H2S(72h)} of cephalexin granules from company A

The C_{H2S(72h)} of cephalexin granules from company A was determined by weighing a sample of 3.5 to 4.5 g of the cephalexin granules into a vial of 20 ml. The H₂S content was analysed as described above under § A.1., with the difference that the sample was equilibrated at an ambient temperature for 72 h. The average value for C_{H2S(72h)} of cephalexin granules from company A was 73 µl of H₂S gas per kg of β-lactam antibiotic.

### Example 1

Comparative experiment A was repeated with the difference that the powder was cooled after drying.

A cooler (Vertical conical mixer, type MCV-3000-N, produced by Bachiller S.A.) was charged with 550 kg cephalexin powder. The wall temperature of the mixer was kept at a temperature of 5 °C. The cephalexin was cooled in the cooler under mixing during 2 hours until a product temperature of just below 15 °C was achieved.

### 1.1. Determination C_{H2S(3h)}

The resulting powder was roller compacted as described above, and the C_{H2S(3h)} of the thus pepared cephalexin granules was determined. Due to the cooling the smell of the resulting product was significantly less intensive.

### 1.2. Determination C_{H2S(72h)}

In addition, the C_{H2S(72h)} of the cephalexin granules as prepared under § 1.1 was determined. A sample of 3.5 to 4.5 g of the cephalexin granules was analysed for the H₂S content according to the method as described under comparative experiment A.2., wherein the sample was equilibrated at ambient temperature (22°C) for 72 h. The average value for C_{H2S(72h)} was 21 µl of H₂S gas per kg of β-lactam antibiotic.

### Comparative experiment B

The C_{H2S(72h)} of cephradine granules from competitor B was determined. A sample of 3.5 to 4.5 g of cephradine granules from competitor B was analysed for the H₂S content according to the method as described under comparative experiment A, wherein the sample was equilibrated at ambient temperature for 72 h.

The average value for C_{H2S(72h)} of cephradine granules from competitor B was 28 µl of H₂S gas per kg of β-lactam antibiotic.

### Example 2

The cephradine wet cake was dried and compacted according to the method as described under comparative experiment A with the difference that after drying the product was cooled as described in Example 1. Subsequently, the C_{H2S(72h)} was determined as described under comparative experiment A.2. The average value for C_{H2S(72h)} was 18 µl of H₂S gas per kg of β-lactam antibiotic.

The results in Examples 1 to 2 show that cooling before compacting results in reduced concentrations of H₂S.

## Claims

1. Process for preparing granules having a bulk density of between 0.4 and 1.0 g/ml comprising a β-lactam antibiotic, said process comprising feeding said β-lactam antibiotic to a roller compactor to form compacts, size reducing, e.g. milling the compacts to produce granules, wherein the temperature of the β-lactam antibiotic that is fed to said roller compactor is below 20°C and wherein C_{H2S(72)} < 50 µl of H₂S gas per kg of β-lactam antibiotic, wherein C_{H2S(72h)} is the volume of H₂S gas above said granules per kg of said β-lactam antibiotic, when a sample of between 3.5 and 4.5 g of said granules is kept in a closed container having a volume of 20 ml at a temperature of 22°C during 72 hours at atmospheric pressure (1 bar).

2. Process for preparing granules having a bulk density of between 0.4 and 1.0 g/ml comprising a β-lactam antibiotic, said process comprising feeding said β-lactam antibiotic to a roller compactor to form compacts, size reducing, e.g. milling the compacts to produce granules, wherein the temperature of the β-lactam antibiotic that is fed to said roller compactor is below 20°C and wherein and wherein C_{H2S(3h)} < 10 µl of H₂S gas per kg of β-lactam antibiotic, when a sample of between 3.5 and 4.5 g of said granules is kept in a closed container having a volume of 20 ml at a temperature of 22°C during 3 hours at atmospheric pressure (1 bar).

3. Process according to any one of claims 1 to 2 wherein said β-lactam antibiotic is a cephalosporin selected from the group consisting of cefaclor, cefadroxil, cephalexin and cephradine or a penicillin selected from the group consisting of amoxicillin and ampicillin.

4. Process according to any one of claims 1 to 3, wherein the process comprises cooling the β-lactam antibiotic prior to said feeding.

5. Granules obtainable by the process according to any one of the claims 1 to 4.

6. Use of an apparatus for the preparation of the granules according to claim 5, said apparatus comprising:
(i) a cooler for cooling an β-lactam antibiotic; and
(ii) a means for compressing the cooled antibiotic,
wherein the cooler is arranged such that the β-lactam antibiotic is cooled to a temperature below 20°C prior to feeding the β-lactam antibiotic to the means for compressing the cooled β-lactam antibiotic.

7. Use according to claim 6, wherein said apparatus further comprises a dryer for drying the β-lactam antibiotic, said dryer arranged such that the dried antibiotic can be fed to the cooler.

## Patentansprüche

1. Verfahren zum Herstellen von Granulat mit einer Schüttdichte zwischen 0,4 und 1,0 g/ml, das β-Lactam-Antibiotikum umfasst, wobei das Verfahren Zuführen des β-Lactam-Antibiotikums zu einem Walzenverdichter zum Bilden von Presskörpern, Größenverringerung, beispielsweise Mahlen der Presskörper zum Herstellen von Granulat, umfasst, wobei die Temperatur des β-Lactam-Antibiotikums, das dem Walzenverdichter zugeführt wird, unter 20 °C beträgt und wobei C_{H2S(72)} < 50 µl an H₂S-Gas pro kg an β-Lactam-Antibiotikum, wobei C_{H2S(72)} das Volumen an H₂S-Gas über dem Granulat pro kg an dem β-Lactam-Antibiotikum ist, wenn eine Probe von zwischen 3,5 und 4,5 g des Granulats in einem geschlossenen Behälter mit einem Volumen von 20 ml bei einer Temperatur von 22 °C über einen Zeitraum von 72 Stunden bei Atmosphärendruck (1 bar) gehalten wird.

2. Verfahren zum Herstellen von Granulat mit einer Schüttdichte zwischen 0,4 und 1,0 g/ml, das β-Lactam-Antibiotikum umfasst, wobei das Verfahren Zuführen des β-Lactam-Antibiotikums zu einem Walzenverdichter zum Bilden von Presskörpern, Größenverringerung, beispielsweise Mahlen der Presskörper zum Herstellen von Granulat, umfasst, wobei die Temperatur des β-Lactam-Antibiotikums, das dem Walzenverdichter zugeführt wird, unter 20 °C beträgt und wobei C_{H2S(3h)} < 10 µl an H₂S-Gas pro kg an β-Lactam-Antibiotikum, wenn eine Probe von zwischen 3,5 und 4,5 g des Granulats in einem geschlossenen Behälter mit einem Volumen von 20 ml bei einer Temperatur von 22 °C über einen Zeitraum von 3 Stunden bei Atmosphärendruck (1 bar) gehalten wird.

3. Verfahren gemäß einem der Ansprüche 1 bis 2, wobei das β-Lactam-Antibiotikum ein Cephalosporin ausgewählt aus der Gruppe bestehend aus Cefaclor, Cefadroxil, Cephalexin und Cephradin oder ein Penicillin ausgewählt aus der Gruppe bestehend aus Amoxicillin und Ampicillin ist.

4. Verfahren gemäß einem der Ansprüche 1 bis 3, wobei das Verfahren Kühlen des β-Lactam-Antibiotikums vor dem Zuführen umfasst.

5. Granulat, erhältlich durch das Verfahren gemäß einem der Ansprüche 1 bis 4.

6. Verwendung einer Vorrichtung zum Herstellen des Granulats gemäß Anspruch 5, wobei die Vorrichtung umfasst:
(i) eine Kühleinheit zum Kühlen eines β-Lactam-Antibiotikums; und
(ii) ein Mittel zum Komprimieren des gekühlten Antibiotikums,
wobei die Kühleinheit derart angeordnet ist, dass das β-Lactam-Antibiotikum vor dem Zuführen des β-Lactam-Antibiotikums zu dem Mittel zum Komprimieren des gekühlten β-Lactam-Antibiotikums auf eine Temperatur unter 20 °C gekühlt wird.

7. Verwendung gemäß Anspruch 6, wobei die Vorrichtung ferner einen Trockner zum Trocknen des β-Lactam-Antibiotikums umfasst, wobei der Trockner derart angeordnet ist, dass das getrocknete Antibiotikum der Kühleinheit zugeführt werden kann.

## Revendications

1. Procédé pour la préparation de granulés ayant une masse volumique apparente comprise entre 0,4 et 1,0 g/ml comprenant un antibiotique β-lactame, ledit procédé comprenant l'introduction dudit antibiotique β-lactame dans un compacteur à rouleaux pour former des comprimés et la réduction de la taille, par exemple le broyage des comprimés pour produire des granulés, dans lequel la température de l'antibiotique β-lactame qui est introduit dans ledit compacteur à rouleaux est au-dessous de 20 °C et dans lequel C_{H2S(72)} est < 50 µl d'H₂S gazeux par kg d'antibiotique β-lactame, C_{H2S(72h)} étant le volume d'H₂S gazeux au-dessus desdits granulés par kg dudit antibiotique β-lactame, lorsqu'un échantillon compris entre 3,5 et 4,5 g desdits granulés est gardé dans un récipient fermé ayant un volume de 20 ml à une température de 22 °C pendant 72 heures à pression atmosphérique (1 bar).

2. Procédé pour la préparation de granulés ayant une masse volumique apparente comprise entre 0,4 et 1,0 g/ml comprenant un antibiotique β-lactame, ledit procédé comprenant l'introduction dudit antibiotique β-lactame dans un compacteur à rouleaux pour former des comprimés et la réduction de la taille, par exemple le broyage des comprimés pour produire des granulés, dans lequel la température de l'antibiotique β-lactame qui est introduit dans ledit compacteur à rouleaux est au-dessous de 20 °C et dans lequel C_{H2S(3h)} est < 10 µl d'H₂S gazeux par kg d'antibiotique β-lactame, lorsqu'un échantillon compris entre 3,5 et 4,5 g desdits granulés est gardé dans un récipient fermé ayant un volume de 20 ml à une température de 22 °C pendant 3 heures à pression atmosphérique (1 bar).

3. Procédé selon l'une quelconque des revendications 1 à 2 dans lequel ledit antibiotique β-lactame est une céphalosporine choisie dans le groupe constitué par le céfaclor, le céfadroxil, la céphalexine et la céfradine ou une pénicilline choisie dans le groupe constitué par l'amoxicilline et l'ampicilline.

4. Procédé selon l'une quelconque des revendications 1 à 3, le procédé comprenant le refroidissement de l'antibiotique β-lactame avant ladite introduction.

5. Granulés pouvant être obtenus par le procédé selon l'une quelconque des revendications 1 à 4.

6. Utilisation d'un appareil pour la préparation des granulés selon la revendication 5, ledit appareil comprenant :
(i) un refroidisseur pour le refroidissement d'un antibiotique β-lactame ; et
(ii) un moyen pour la compression de l'antibiotique refroidi,
le refroidisseur étant conçu de façon à ce que l'antibiotique β-lactame soit refroidi à une température au-dessous de 20 °C avant introduction de l'antibiotique β-lactame dans le moyen pour la compression de l'antibiotique β-lactame refroidi.

7. Utilisation selon la revendication 6, ledit appareil comprenant en outre un sécheur pour le séchage de l'antibiotique β-lactame, ledit sécheur étant conçu de façon à ce que l'antibiotique séché puisse être introduit dans le refroidisseur.
